# EUROPÄISCHE PATENTANMELDUNG

(11) **EP 1 870 826 A1**
(43) Veröffentlichungstag der Anmeldung: **26.12.2007**
(21) Anmeldenummer: 06013048.1
(22) Anmeldetag: 23.06.2006
(51) Int. Cl.: G06F 19/00

(54) **Notfalleinrichtung zur Anzeige von Patientendaten**

(71) Anmelder: Pharmatechnik GmbH & Co. KG, 82319 Starnberg (DE)
(72) Erfinder: Graessner, Detlef, 82131 Gauting (DE)
(74) Vertreter: Becker Kurig Straus

(57) **Zusammenfassung**

Ein Verfahren zum Betreiben einer Notfalleinrichtung umfasst die Phasen eines Bereitstellens und Zusammenführens von Quelldaten eines Warenwirtschaftssystem und eines Patientendatensystems, eines Filterns der Quelldaten für eine Schnittstelle nach Ersthilfekriterien zur Aufbereitung und Formung notfallrelevanter Daten eines Patienten. Das Verfahren umfasst weiter die Phasen eines Umwandelns der notfallrelevanten Daten in Zieldaten, die für in eine mobile Ersthilfeapplikation geeignet sind, eines Kommunizierens der Zieldaten in eine mobile Ersthilfeapplikation mittels Übertragung, eines Einbindens der Zieldaten in einen Kontext der mobilen Ersthilfeapplikation. Schließlich umfasst das Verfahren zum Betreiben einer Notfalleinrichtung die Phasen eines Vorsehens einer notfallgeeigneten Abruf- oder Anzeigemöglichkeit der Zieldaten durch eine Notfalltaste einer mobilen Ersthilfeapplikation und eines selektiven Zurverfügungstellens der Zieldaten auf dem mobilen Endgerät im Wege einer formatierten Ausgabe. Dies ermöglicht oder erleichtert dem behandelnden Notfallarzt an der Einsatzstelle die Diagnose und Einleitung geeigneter Ersthilfemaßnahmen.

## Beschreibung

Die vorliegende Erfindung betrifft eine Notfalleinrichtung, insbesondere eine Einrichtung die auf der Basis eines mobilen Gerätes, welches geeignete Informationen und Kommunikationsmöglichkeiten bereithält, um die Rettungschancen bei einem Unfall wesentlich verbessern bzw. um die Erstversorgung durch z.B. einen Notarzt zu erleichtern.

In einem möglichen Szenario ist eine Person z.B. ohne Papiere auf einem Fahrrad im Wald und ländlichem Gebiet unterwegs und erleidet plötzlich einen Schlaganfall und stürzt zu Boden. Die Person verliert ihre Orientierung und ist bewusstlos. Glücklicherweise kommt jedoch ein Passant des Weges und es gelingt ihm einen Notarzt zu rufen. Dem nach einiger Zeit eintreffenden Notarzt gelingt zwar die Wiederbelebung, die Person ist aber verwirrt und der Arzt weiß nichts über die fremde Person, die sich nicht artikulieren kann und unter Schock steht. Insbesondere weiß der Arzt nichts über den Gesundheitszustand und die medizinische Vorgeschichte. Der Arzt weiß insbesondere weder, ob bei der Person chronische Probleme wie Diabetes, Bluthochdruck, Allergien vorliegen, noch weiß er über die verwendeten Medikamente, Unverträglichkeiten bescheid. Zwar mag es dem Arzt unter Ansehung der Risiken irreparabler Schäden gelingen, über äußere Merkmale eine einfache Vorortdiagnose durchzuführen, aber hier würde eine falsche Behandlung fatale Folgen haben. Daher wird die Person nur stabilisiert und zur weiteren Untersuchung ins Krankenhaus gebracht, sofern das möglich ist und kein weiterer Unfall geschieht. Während des Transports und der anschließenden Untersuchung vergeht wertvolle, vielleicht zu viel Zeit und es entstehen vermeidbare Kosten. Auch werden die Angehörigen oder Geschäftspartner des Patienten erst mit Verspätung benachrichtigt werden und können nicht entsprechende Maßnahmen oder Verfügungen treffen, was den Verunglückten zusätzlich belastet und weitere vermeidbare Kosten entstehen.

Daher wäre es wünschenswert, wenn der behandelnde Arzt einen Informationsvorteil und eine Entscheidungshilfe hätte, der es gestattet, die auftretenden Therapierisiken zu verringern.

Bekannte Lösungen, wie in der DE 198 29 377 C2 vorgeschlagen, sehen eine Notfallalarmierungsvorrichtung vor, bei der die Person eine Uhr am Handgelenk trägt, die eine Sendeeinrichtung enthält, die bei drohender Gefahr oder Gewalteinwirkung einen Notfallcode sendet. Nachteilig ist an dieser Lösung jedoch, dass die Uhr bei dem vorliegenden Szenario durch den Sturz beschädigt sein kann und sich ohnehin nur schwer bedienen lässt.

Weiter ist aus der DE 196 07 157 eine ähnliche, körpernah getragene Vorrichtung zur Ortung und Markierung von Verschütteten bekannt, die auch sensorisch bestimmte Körperfunktionen erfasst und sendet. Jedoch ist hier eine vom Bergungspersonal mitgetragene Empfangsvorrichtung vorgesehen, wobei es im vorliegenden Szenario aber erforderlich wäre, dass der Arzt Zusatzwissen über die von der Person mitgeführte Uhr hätte und den passenden Empfänger.

Daneben gibt es eine Kombination aus Messwerterfassungs-, Kommunikations- und Ortungseinrichtung, die es gestattet den Gesundheitszustand zu überwachen und ggf. automatisch Hilfsdienste zu benachrichtigen, wenn ein Notfall eingetreten ist, mit der Möglichkeit die Peson zu finden und erste Hilfe- oder lebenserhaltende Maßnahmen so bald wie möglich einzuleiten.

Für ähnliche Zwecke offenbart die WO 00/16288 ein Schutz- und Überwachungssystem für hilfebedürftige Personen, indem ein auf dem globalen Positionierungssystem (GPS) beruhendes Verfahren verwendet wird, wobei jedoch keine physikalische Überwachung vorgesehen ist.

Aus der DE 198 44 296 ist eine Gerät für eine Patientenüberwachung bekannt, das aus einem Sensor für physiologische Parameter und einem Signalprozessor, wie auch einem mobilen Endgerät für eine Kommunikation in einem zellularen Mobilkommunikationsnetzwerk besteht. Dieses ist in der Lage, eine zellenbasierte Ortung des mobilen Endgerätes auf der

Basis der verwendeten Basisstation durchzuführen.

Auch offenbart die WO 03/041290 ein mobiles Telefon mit einer Alarmfunktion, bei der eine Notfalllampe aktiviert wird und ein Alarm erzeugt wird, wenn ein Alarmknopf gedrückt wird. Dabei kann der Herzschlag des Benutzers durch ein enthaltenes Stethoskop gemessen werden.

Daneben wird in der Anmeldung DE 196 39 492 ein automatisches Hilfeaktivierungssystem offenbart, das ein Mobiltelefon und ein GPS-System, wie auch bestimmte Messungs- und Erfassungsvorrichtungen umfasst. Dieses bestimmt seine Position über GPS und aktiviert sich, wenn bestimmte Kriterien erfüllt sind.

Zwar gelingt es bei dem vorgenannten Stand der Technik, die Alarmierung des Arztes nach vorne zu verlagern, auch mag er über gewisse messtechnisch erfasste oder aktuell übertragene Größen einen gewissen Vorteil und eine Einschätzung über den Gesundheitszustand haben.

Fraglich ist jedoch, ob dies im vorliegenden Szenario die Rettungsaussichten verändert oder verbessert, da der Arzt immer noch nicht den wünschenswerten Informationsvorteil hat hinsichtlich eingesetzter Therapiemittel, insbesondere Medikamente oder Unverträglichkeiten. So wäre es vorteilhaft, wenn der Arzt sich Vorort über den Patienten über Anschrift, Blutgruppe, eingenommene Arzneimittel, wie z.B. Adalate, Diabetesmittel, Medikamente etc., informieren kann. Weiterhin sollten Personen, die direkt kontaktiert werden können, wie der Hausarzt, Krankenkasse, oder die Verwandten ohne größeren Aufwand recherchierbar sein.

Die Aufgabe der vorliegenden Erfindung besteht darin eine Notfalleinrichtung bereitzuhalten, die dem Arzt den entscheidenden Informationsvorteil zur verbesserten Erstdiagnose verschafft. Weiterhin sollte mit einer geeigneten Vorrichtung erstmals ein Ersatz für die Gesundheitskarte geschaffen werden.

Diese Aufgabe wird durch das "Erste deutsche Apothekenfunktelefon" und ein darauf gerichtetes Verfahren gelöst, welches eine SOS-Funktionalität aufweist, die besonderes für Notfälle geeignet ist.

In einer Ausführungsform sind auf dem Datenspeicher eines Funktelefons Patientendaten wie beispielsweise die aktuelle Medikation, Unverträglichkeiten. Blutgruppe, Krankheiten, Adressen von Angehörigen, dem Hausarzt und der Krankenkasse verfügbar. Diese Daten werden geeigneterweise auf einer Bildschirmoberfläche, welche eine SOS-Taste, beispielsweise einen sogenannten Softkey, bereithält, in einer Maske angezeigt oder auf einem Drucker ausgegeben werden. Weiter ist eine Kommunikationsmöglichkeit zur Kontaktaufnahme mit den dort gespeicherten Personen möglich.

In einer weiteren Ausführungsform ist ein Verfahren vorgesehen, das ein Beispiel zeigt, wie in dem eingangs beschrieben Szenario die SOS-Funktionalität als Handgerät-basierte Anwendung realisiert ist.

Die begleitenden Zeichnungen werden eingeschlossen, um ein weiteres Verständnis der Erfindung zu liefern, werden Bestandteil der Erfindung und konstituieren einen Teil dieser Beschreibung. Die nachfolgenden Zeichnungen erläutern Ausführungsformen der vorliegenden Erfindung und dienen zusammen mit der Beschreibung dazu, die Prinzipien der Erfindung zu erklären. Es zeigen:
- Fig. 1: ein mobiles Endgerät mit einer Benutzeroberfläche und einer SOS-Funktionalität gemäß einer Ausführung der vorliegenden Erfindung;
- Fig. 2: eine Ausgabe der Patientendaten durch eine mobile Applikation auf einem Endgerät;
- Fig. 3: ein Flussdiagramm für einen Verfahrensablauf einer SOS-Funktionalität; und
- Fig. 4: eine Prozessdarstellung für eine Ersthilfeapplikation gemäß einer alternativen Ausführung der vorliegenden Erfindung.

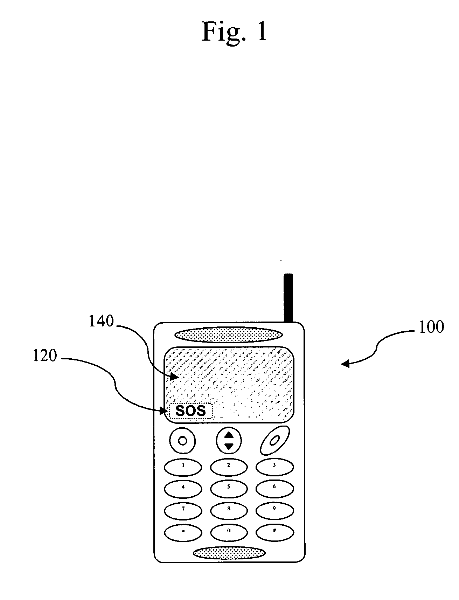

Fig. 1 zeigt ein Mobiles Endgerät mit einer SOS-Funktionalität. In dem mobilen Endgerät, hier beispielshalber als Mobiltelefon 100 abgebildet, das typischerweise einen Prozessor, einen Speicher und eine Anzeigeeinheit 140 aufweist.

Auf der Anzeige 140, beispielsweise ein mobiler Farbbildschirm, der eine mobile Benutzeroberfläche aufweist, ist eine weiche Taste 120 vorgesehen, welche die Aufschrift SOS trägt und sich beispielsweise in einer Warteposition am linken unteren Bildrand der Anzeige befindet. Alternativ kann eine belegbare mechanische Taste, die beleuchtbar unterlegt werden kann, vorgesehen werden. Diese Taste kann alternativ auch ein Notfallsymbol tragen.

Dabei kann die Anzeigeeinheit 140 des mobilen Endgerätes 100 eine berührungsempfindliche oder bewegungsantwortend abgetastete oder berührbare Oberfläche aufweisen, Zeige-, Stift- oder Navigationsmittel bereithalten, mit der die Taste betätigt werden kann. Bei der SOS-Taste kann es sich beispielsweise um ein kleines aber häufig abgefragtes Unterprogramm handeln, das die Taste bereitstellt.

Wird die Taste durch Drücken oder Berühren aktiviert, kann die Taste eine vergrößerte Form einnehmen, rot leuchten, blinken und über einen voreinstellbaren Klang den Notfall signalisieren. Über ein internes Nachrichtensystem des Betriebssystems kann sodann eine Verbindung zu der erfindungsgemäßen Ersthilfeapplikation aufgerufen werden, welche dann die Steuerung übernimmt und mit der Ausführung eines Notfallplanes oder der Anzeige der notfallrelevanten Daten beginnt.

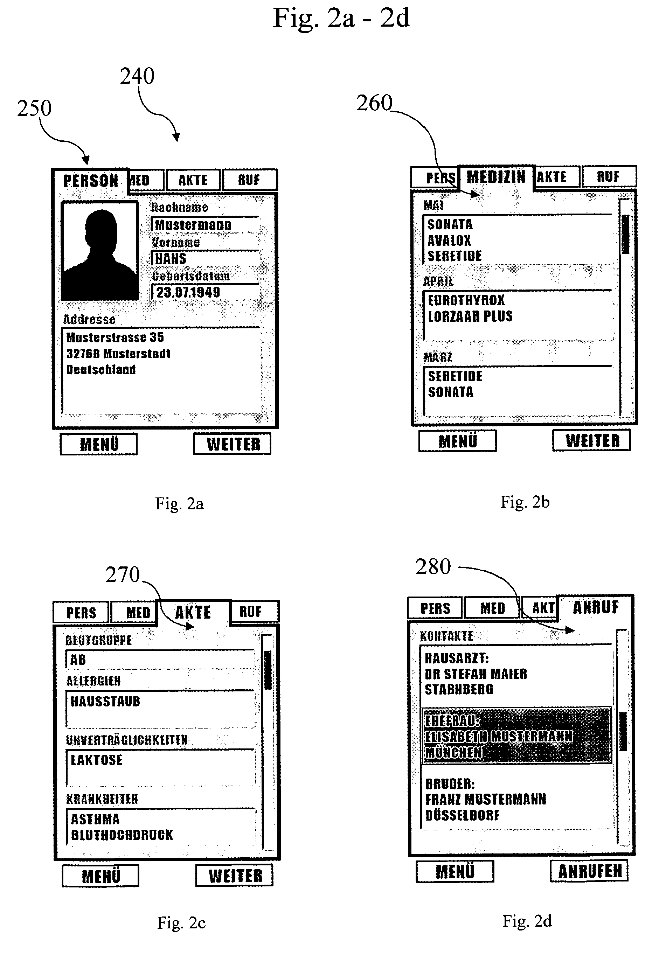

Fig. 2a bis 2d zeigen eine formatierte Datenausgabe auf einem Bildschirm 240 des mobilen Endgerätes. Auf der ersten Seite 250 sind ein Bild und die Daten der Person, Name, Vorname und Geburtsdatum gespeichert wie auch die Adresse. Auf der zweiten Seite 260 ist eine Aufteilung der erworbenen Präparate oder Medikamente in den letzten Monaten vorgesehen. Auf der Seite 270 werden bestimmte Patientendaten wie Blutgruppe, Allergien, Unverträglichkeiten und Krankheiten angezeigt. Auf der nächsten Seite 280 werden dann Kontakte angezeigt, die angerufen werden können, zum Beispiel nahe Angehörige, der Hausarzt und die Krankenkasse (nicht gezeigt).

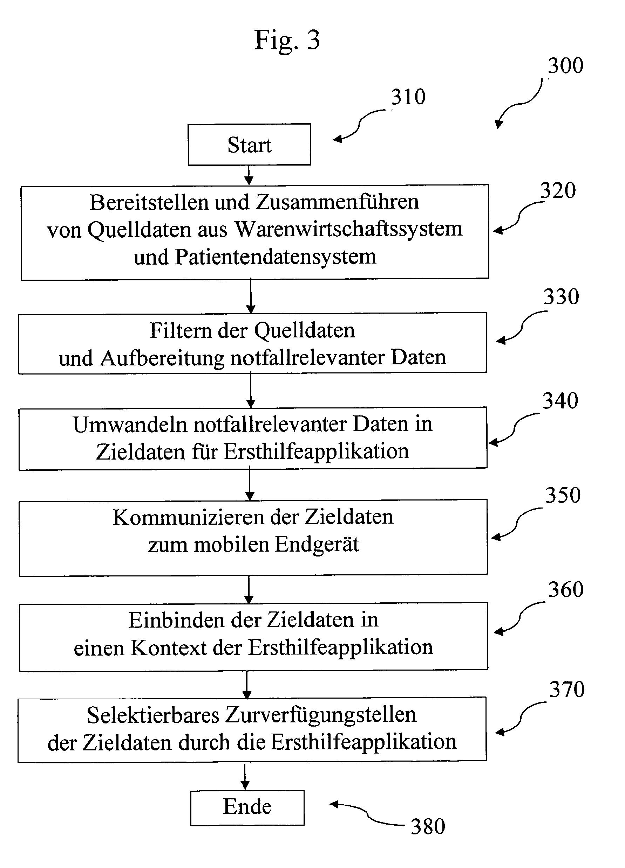

Fig. 3 zeigt den Ablauf eines Verfahrens zur Anzeige notfallrelevanter Daten. Nach dem Start 310 können in einer Anfangsphase 320 des Verfahrens 300 Quelldaten aus einem Warenwirtschaftssystem gewonnen, und/oder entnommen werden und mit Quelldaten aus einem Patientendatensystem zusammengeführt werden. Es entsteht eine erfindungsgemäße Datenbasis. Das Warenwirtschaftssystem und Patientendatensystem werden als Datenminen iSv Datenvorkommen betrachtet. Die Zusammenführung kann über erprobte relationale Datenbankkriterien erfolgen.

In einer weitem Phase 330 können die Quelldateien nach besonderen Kriterien gefiltert werden, die für eine Ersthilfe von besonderer Bedeutung sind. Die Filterung kann dabei über eine verschachtelte Anzahl an Filterregeln oder Einstellungen gehen und eine Gewichtung mehrerer oder die Ersetzung nicht vorhandener Werte durch nächstkommende oder Standartdaten vornehmen. Aus den gefilterten Quelldaten werden sogenannte notfallrelevante Daten extrahiert. Dabei werden unter notfallrelevanten Daten solche hoher Qualität verstanden, in der Regel sind das wichtigste Daten mit hohem Informationswert. Derartige Daten sind im Notfall lastfest, derart, dass sie für einen Arzt vor Ort während der Feuerprobe der kritischen Situation zur Entscheidung über einzuleitende Diagnosen oder Maßnahmen als Anregung oder Entscheidungshilfe wirksam herangezogen werden können.

Dabei versteht es sich, dass für die Gewinnung von notfallrelevanten Daten weniger typische Datenbankfähigkeiten herangezogen werden können, vielmehr stehen dahinter Expertensysteme, Supercomputer oder neuronale Netzwerke mit im wesentlichen endlicher Rechenleistung, denen es erfindungsgemäß gelingt, eine optimale Abbildung der Quelldaten im Wege der Datenveredelung in die Zieldaten zu ermöglichen.

Hierbei können auch Rückfragen an die Datenminen erforderlich sein, die eine Kommunikation mit der Datenbasis oder einer der Datenminen umfassen, wobei besondere Historie des einzelnen Datensatzes oder einer Gruppe von ähnlichen Datensätzen berücksichtigt werden kann.

In einer vorteilhaften Ausführungsform kann in einer ähnlichen oder anderen Phase, die sich an eine Veredelung als zweite Stufe im Sinne einer Nachveredelung anschließen kann, wobei die Phase aber auch die direkt aus den Datenminen heraus angestoßen bzw. in sie eingetreten werden kann, das Filtern der Quelldaten für eine Schnittstelle nach Ersthilfekriterien zur Aufbereitung und Formung notfallrelevanter Daten eines Patienten vorgenommen werden.

In diesem Fall werden unter einer Schnittstelle eine Datenstruktur verstanden, als verknüpfte Liste, als einfacher Komma-trunkierter Datensatz, ein Paketdatensatz oder ähnliches, wie auch ein Objekt, das die Möglichkeit einzelner Datenoperationen unterstützt. Üblicherweise werden über diese Schnittstelle die Daten übertragen, dabei können sie jedoch modifiziert, und/oder transformiert werden. Dabei versteht es sich, dass auch eine verteilte, auf mehreren Systemen laufende Anwendung die einzelnen Phasen interaktiv, oder soweit möglich, parallel ausführen kann.

In einer weiteren Phase 340, kann ein Umwandeln der notfallrelevanten Daten in Zieldaten vorgesehen sein, wobei die Zieldaten für in eine mobile Ersthilfeapplikation geeignet sind. Hierbei werden die veredelten Daten derart formatiert, dass sie in die Umgebung einer mobilen Applikation eingebettet werden können. Dies kann je nach Ausgestaltung, dem gewählten Übertragungsweg, und/oder der gewählten Schnittstelle eine Vielzahl an Umwandlungsphasen aufweisen. Die Umwandlung kann jedoch insofern erfindungsgemäß besonders ausgestaltet sein, als die Konsistenz der Daten transformations-invariant gewährleistet sein muß, damit die Lastfestigkeit der notfallrelevanten Daten erhalten bleibt.

In einer Phase 350, die sich an die Datengewinnung oder über eine Datenveredelung anschließt, kann ein Kommunizieren der Zieldaten in eine mobile Ersthilfeapplikation im Wege einer Übertragung erfolgen. Dabei kann das Kommunizieren einen uni- oder bidirektionalen, gesicherten oder wiederholten Datentransfer vorsehen, sei es über mehrere Schnittstellen, Übertragungswege oder Computer bzw. Netze hinweg, wobei besondere Vorkehrungen zur Sicherung und zum Konsistenzerhalt der notfallrelevanten Daten getroffen werden können. Aufgrund der Notfallrelevanz der Daten können auch bestimmte Übertragungswege, Verbindungsprotokolle bevorzugt sein.

In einer weiteren Phase 360, die sich an die Übertragung anschließen kann, kann ein Einbinden der Zieldaten in einen Kontext der mobilen Ersthilfeapplikation erfolgen. Im einfachsten Falle, werden die Zieldaten bestimmten Datenfeldern und/oder Speicherbereichen der mobilen Applikation, die für ein mobiles Endgerät geeignet ist, zugeordnet.

Daneben ist in einer anderen Phase ein Vorsehen einer notfallgeeigneten Abruf- oder Anzeigemöglichkeit der Zieldaten im Wege einer Menü-Funktionalität in der mobilen Ersthilfeapplikation beabsichtigt.

Erfindungsgemäß ist eine sogenannte SOS-Funktionalität vorgesehen. Dabei handelt es sich um eine Funktion, die über einen Programmpunkt, oder eine sogenannte weiche Taste ausgeführt werden kann, insbesondere wie in Fig. 1 beispielhaft veranschaulicht.

Da es sich um eine notfallkritische Funktion handelt, können besondere Ausführungsformen vorsehen, die Bediendung so zu unterstützen, dass auch eine weitere in der Wahrnehmung eingeschränkte, verwirrte oder gar auch unter Schock stehende Person oder ein kleines Kind die Funktion noch bedienen kann, um einen Arzt zu rufen.

Die notfallkritische Applikation kann einen Notfallplan vorsehen, bei dem verschiedene Abläufe voreingestellt werden, so dass automatische Sprach- oder Textnachrichten, Lageplan des Ortes oder geographische Koordinaten des Unfalls versandt werden, falls der Benutzer nicht innerhalb einer kurzen Zeitspanne bestimmte Fragen beantwortet oder Menüpunkte ausführt, insbesondere falls der Arzt noch nicht zur Stelle ist.

In einer nächsten Phase 370 erfolgt ein selektives Zurverfügungstellen der Zieldaten auf dem mobilen Endgerät im Wege einer formatierten Ausgabe. Erfindungsgemäß ist es vorgesehen, dass die vorbereiteten und veredelten notfallrelevanten Daten im Notfall von Rettungskräften in kürzester Zeit mit maximaler Wirkung aufgerufen und abgelesen werden können.

Weiter kann es vorgesehen werden, dass aus der Ersthilfeapplikation direkt die wichtigsten Verbindungen aufgebaut werden, wobei hier besondere Teile des Betriebssystems aktiviert oder umgangen werden können.

Das Verfahren endet in Phase 380, falls zum Beispiel eine der Tasten "Menü" oder "Weiter" gedrückt wird, wie in Fig. 2 gezeigt.

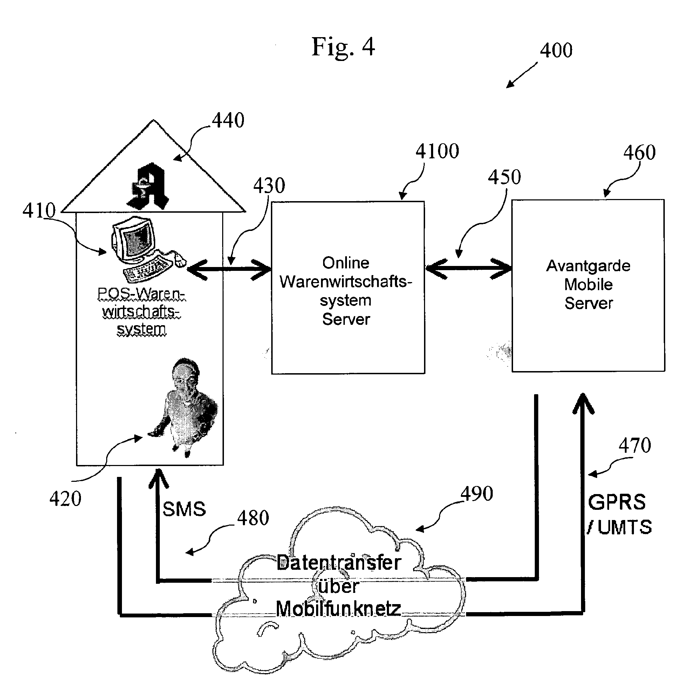

In Fig. 4 wird eine vereinfachte Prozessdarstellung 400 für eine Ersthilfeapplikation dargestellt, wie sie einer alternativen Ausführung der vorliegenden Erfindung ablaufen kann.

In einer vorteilhaften Ausführungsform weist eine Apotheke 440 einem Verkaufsstellenpunkt 410 mit einem Warenwirtschaftssystem auf, das mit einer Datenmine, zum Beispiel einem Server 4100 in Verbindung steht. Dort befindet sich ein Benutzer eines mobilen Endgerätes 420 mit einer mobilen Applikation. Über eine erste Internetverbindung 430 können Daten von dem lokalen Warenwirtschaftssystem 410 an einen Server 4100 eines Warenwirtschaftssystem übertragen werden, welcher mehrere Apotheken 440 betreuen kann. Diese und andere Daten können über eine zweite Internetverbindung 450 an einen weiteren Server 460 eines Dienstleisters geeignet nach einem Verfahren der vorliegenden Erfindung, zum Beispiel Phase 330, bearbeitet und weitergereicht werden. Daneben kann das mobile Endgerät 420 bzw. die dort enthaltende mobile Applikation über eine Nachricht eines Kurznachrichtendienstes (SMS), die über eine Verbindung 480 übertragen wird, gesteuert aktiviert bzw. aufgeweckt werden. Weiter können von dem mobile Endgerät 420 über eine generelle Paketradioverbindung (GPRS) und/oder über eine Verbindung eines universalen mobilen Transfersystems (UMTS) 470 von dem Server 460 Daten durch mobile Applikation heruntergeladen, verarbeitet und gespeichert werden. Es versteht sich, dass die hier in einer Richtung angegebene Kommunikation zwischen dem mobile Endgerät 420 und dem Server 460 des Dienstleisters über das wolkenförmig dargestellte Mobilfunknetz 490 auch in beide Richtungen erfolgen kann. Alternativ kann das Mobilfunknetz auch durch ein Festnetz oder ein Kommunikationsnetz anderer Art, Internet, lokales oder Weitbereichsnetzwerk ersetzt werden, so dass eine dritte Internetverbindung besteht.

Übrigens kann zwischen dem mobile Endgerät 420 und dem lokalen Warenwirtschaftssystem 410 über geeignete Schnittstellen ein Datenaustausch über Nahbereichskommunikationsmittel, oder Netzwerkverbindungen als eine vierte Verbindung (nicht gezeigt)auf optischer oder hochfrequenter Basis stattfinden, so dass eine sog. geschlossene Ringkommunikation möglich ist. Der zwischenverbundene Ring wird dabei durch das Warenwirtschaftssystem 410, den Server 4100, den Dienstleisterserver 460, die das Netz 490 und das mobile Endgerät 420 gebildet. Damit gelingt es, die Übertragungssicherheit und die Datenkonsistenz der notfallrelevanten Daten mit echtzeitnahem Bezuge sicherzustellen. Weiterhin kann erstmals ein Ersatz für die Gesundheitskarte geschaffen werden, als diese durch den Speicher oder eine Speichervorrichtung im mobilen Endgerät verwirklicht werden kann, wobei die Daten im Speicher schnell aktualisiert werden können.

## Patentansprüche

1. Verfahren zum Betreiben einer Notfalleinrichtung, die folgende Phasen umfasst :
Bereitstellen und Zusammenführen von Quelldaten aus einem Warenwirtschaftssystem und einem Patientendatensystem;
Filtern der Quelldaten für eine Schnittstelle nach Ersthilfekriterien zur Aufbereitung und Formung notfallrelevanter Daten eines Patienten;
Umwandeln der notfallrelevanten Daten in Zieldaten, die für in eine mobile Ersthilfeapplikation geeignet sind;
Kommunizieren der Zieldaten in eine mobile Ersthilfeapplikation im Wege einer Übertragung ;
Einbinden der Zieldaten in einen Kontext der mobilen Ersthilfeapplikation;
Vorsehen einer notfallgeeigneten Abruf- oder Anzeigemöglichkeit der Zieldaten im Wege einer Menü-Funktionalität in der mobilen Ersthilfeapplikation; und
Selektives Zurverfügungstellen der Zieldaten auf dem mobilen Endgerät im Wege einer formatierten Ausgabe.

2. Verfahren gemäß Anspruch 1, bei dem man Phasen einer notfallgeeigneten Abrufmöglichkeit der Zieldaten durch eine SOS-Taste einer mobilen Applikation vorsieht.

3. Verfahren gemäß Anspruch 1, bei dem man Phasen einer notfallgeeigneten Anzeigemöglichkeit der Zieldaten durch eine Applikation mit mehreren Masken vorsieht.

4. Notfalleinrichtung, die umfasst :
ein mobiles Endgerät, mit einem Speicher und einem Prozessor zur Ausführung des Verfahrens nach einem der vorhergehenden Ansprüche, zusätzlich aufweisend eine Notfalltaste.

5. Notfalleinrichtung gemäß Anspruch 4, bei dem in dem Speicher des Mobilfunkgerätes Daten einer Gesundheitskarte abgebildet werden und/oder enthalten sind.

6. Computerprogrammprodukt, das einen Programmcode aufweist, welcher entweder in der Form eines maschinenlesbaren Mediums vorliegt, bei dem ein Programmcode auf dem Medium gespeichert ist, oder welches in der Form eines ausbreitungsfähigen, evaneszenten, oder nicht abstrahlenden Signales vorliegt, das eine Repräsentation eines Programmcodes umfasst,
wobei der Programmcode angeordnet ist, dass er ein Verfahren gemäß einem der vorhergehenden Ansprüche ausführt, wenn es auf einem auf einer eingebetteten Steuerung, einem Computersystem, einem tragbaren Computer, einem Personalen Datenbegleiter oder -assistenten, Smart-Telefon abgearbeitet wird, wenn der Klient, der Server, das Modul nach einem Ladevorgang einer Datenstruktur in einen Arbeits- oder Hauptspeicher eines Rechners oder einer Mehrzahl an Rechnern eines Computernetzwerkes oder einem Betreiben eines Klienten in einem Klienten Servernetz ausführt.

7. Datenstruktur, die einen Algorithmus für eine SOS-Funktionalität gemäße einem der vorhergehenden Ansprüche aufweist.

8. Computerlesbares Medium, auf welchem eine Datenstruktur gespeichert wird, wobei die Datenstruktur das Verfahren gemäß einem der vorhergehenden Anspruche, nach einer Ladeoperation der Datenstruktur in einen Arbeits- oder Hauptspeicher eines Computers oder einer Mehrzahl an Computer in einem Netzwerk ausführt.

9. Moduliertes Datensignal, Computerdatensignal oder spektrale Signatur, die in einer Trägerwelle verkörpert ist, welche aus ausführbaren Instruktion zum Ausführen eines Verfahrens auf einem Computersystem eines Computernetzwerkes oder einer Mehrzahl an Computern eines Computernetzwerkes gemäß einem der vorhergehenden Ansprüche besteht.

## Geänderte Patentansprüche

### Geänderte Patentansprüche gemäss Regel 137(2) EPÜ.

**1.** Verfahren zur Notfall-Benachrichtigung für ein Mobiltelefon, wobei das Verfahren in Reaktion auf eine Aktivierung einer Notfall-Vorrichtung des Mobiltelefons eingeleitet wird, umfassend:
Bestimmen von Standortdaten des Mobiltelefons;
Bestimmen von Patientendaten;
automatisches Filtern der abgerufenen Patientendaten, wobei die Filterregeln für das automatische Filtern eine Gewichtung der abgerufenen Patientendaten nach medizinischen Kriterien umfassen;
Zusammenfügen der gefilterten Patientendaten und der Standortdaten in ein Notfall-Datenpaket;
Umwandeln des Datenpakets in Zieldaten, die kompatibel zu verschiedenen medizinischen Gerätestandards sind;
Senden des Notfall-Datenpakets an eine Rettungs-Leitstelle.

**2.** Verfahren gemäß Anspruch 1, wobei das Bestimmen von Standortdaten ein Verwenden von GPS-Signalen, Galileo-Signalen, GLONASS-Signalen, Compass-Signalen, Signalen von WLAN-Hotspots, Signalen von Mobilfunk-Basisstationen oder Kombinationen daraus umfasst.

**3.** Verfahren gemäß Anspruch 1, wobei das Bestimmen von Patientendaten ein Abrufen von lokal gespeicherten medizinisch relevanten Daten umfasst, die auf einer Speicher-Vorrichtung des Mobiltelefons gespeichert sind.

**4.** Verfahren gemäß Anspruch 1, wobei das Bestimmen von Patientendaten ein Abrufen von entfernt gespeicherten, medizinisch relevanten Daten umfasst.

**5.** Verfahren gemäß Anspruch 1, **gekennzeichnet dadurch, dass** die Zieldaten auf dem Mobiltelefon angezeigt werden.

**6.** Verfahren gemäß Anspruch 3 oder 4, wobei die Patientendaten nach einer autorisierten Anforderung durch externe Geräte abgerufen werden, die in der Lage sind, mit dem Mobiltelefon zu kommunizieren.

**7.** Mobiltelefon umfassend:
mindestens eine Vorrichtung zur drahtlosen Kommunikation;
Speicher-Vorrichtung;
Prozessor;
Empfänger-Vorrichtung, die in der Lage ist drahtlose Signale zu empfangen, mit denen eine Positionsbestimmung durchgeführt werden kann;
wobei das Mobiltelefon **dadurch gekennzeichnet ist, dass**:
das Mobiltelefon eine Notfalltaste umfasst, durch deren Aktivierung ein Verfahren zur Notfall-Benachrichtigung eingeleitet und ausgeführt wird, und
der Prozessor eingerichtet ist, ein Verfahren zur Notfall-Benachrichtigung auszuführen.

**8.** Mobiltelefon gemäß Anspruch 7, wobei das Mobiltelefon eine AnzeigeVorrichtung zur Darstellung von Informationen auf dem Mobiltelefon umfasst.

**9.** Mobiltelefon gemäß Anspruch 7, umfassend Mittel zur Bestimmung von Standortdaten.

**10.** Mobiltelefon gemäß Anspruch 7, umfassend Mittel zur Bestimmung von Patientendaten.

**11.** Mobiltelefon gemäß Anspruch 7, wobei der Prozessor eingerichtet ist, ein Verfahren zur Notfall-Benachrichtigung gemäß Anspruch 1 in Reaktion auf eine Aktivierung der Notfall-Vorrichtung auszuführen.

**12.** Mobiltelefon gemäß Anspruch 7, wobei das Mobiltelefon eine Abruf-Vorrichtung umfasst, die es einem externen Gerät erlaubt, Informationen von dem Mobiltelefon abzurufen, die auf dem Mobiltelefon gespeichert sind ohne dass die Notfalltaste betätigt wurde.

**13.** Mobiltelefon gemäß Anspruch 7, wobei die Speicher-Vorrichtung eine Wechsel-Speicher-Vorrichtung ist.

**14.** Mobiltelefon gemäß Anspruch 7, wobei die Speicher-Vorrichtung die Standards einer Gesundheitskarte erfüllt.

**15.** Computerprogramm-Produkt, das Computer-Programm-Code-Mittel umfasst, die geeignet sind, die Schritte des Verfahrens von Anspruch 1 bis 6 auszuführen.

**16.** Computerprogramm-Produkt gemäß Anspruch 15, das in ein computerlesbares Medium eingegliedert ist.
